# EUROPÄISCHE PATENTSCHRIFT

(11) **EP 0 939 736 B1**
(45) Veröffentlichungstag und Bekanntmachung des Hinweises auf die Patenterteilung: **25.04.2001**
(21) Anmeldenummer: 97954812.0
(22) Anmeldetag: 07.11.1997
(51) Int. Cl.: B65G 47/14

(54) **TABLETTENPRÜFGERÄT**
TABLET TESTING DEVICE
APPAREIL A CONTROLER LES COMPRIMES

(30) Priorität: 08.11.1996 US 29889 P
(43) Veröffentlichungstag der Anmeldung: 08.09.1999
(73) Patentinhaber: Dr. Schleuniger Pharmatron AG, 4501 Solothurn (CH)
(72) Erfinder: BRACHER, Martin, CH-4501 Solothurn (CH)
(74) Vertreter: Rosenich, Paul
(86) Internationale Anmeldenummer: EP9706200
(87) Internationale Veröffentlichungsnummer: WO9819945

(56) Entgegenhaltungen:
- WO-A-85/03278
- DE-A- 3 711 827
- DE-U- 8 911 221
- US-A- 4 784 275
- US-A- 5 240 118
- US-A- 5 522 512

## Beschreibung

Die Erfindung betrifft ein Tablettenprüfgerät.

Tablettenprüfgeräte sind Vorrichtungen, die hauptsächlich in der Pharmaindustrie oder in Wissenschaft und Forschung angewendet werden. Da es bei Medikamenten, die in Tablettenform verabreicht werden, zum Teil sehr präzise Qualitäts- und Quantitätsanforderungen gibt, werden wenigstens stichprobenweise bestimmte Parameter der jeweiligen Tabletten gemessen.

Bekannt sind für solche Messvorgänge Geräte, die mehrere Prüf- bzw. Messstationen beherbergen und bei denen Tabletten aus einem Vorratsbehälter zu den einzelnen Stationen gefördert werden. Ein Beispiel für ein solches Gerät ist das Tablettenkombinationsprüfgerät "Multicheck" der Firma ERWEKA D-63150 Heusenstamm. Es ermöglicht das automatische bzw. computergesteuerte Messen von Gewicht, Dicke, Durchmesser und Bruchfestigkeit einzelner Tabletten, die nacheinander aus einem Vorratsbehälter angeliefert werden sollen.

Gerade das vereinzelte Zuführen von Tabletten ist bei solchen Geräten jedoch ein Problem. So versucht gerade die erwähnte Firma mittels EP-B1-170670 eine besonders geeignete Transporteinrichtung für Tabletten innerhalb eines solchen Tablettenkombinationsprüfgerätes zu beschreiben. Die Transporteinrichtung ist so aufgebaut, dass ein Rechen mit V-förmigen Gabeln die Tabletten über eine Führungsbahn verschiebt, die genau dimensionierte Löcher aufweist, durch die nicht passende Tabletten fallen sollen. Diese Vorrichtung bildet somit eine Art Sieb, wobei es davon abhängt, wie gross die Tabletten sind und wo sie an den Gabeln (zufällig) anliegen, ob sie weitergefördert werden oder durch die Löcher fallen. Es bleibt somit eine Unsicherheit bestehen, die zu Messverzögerungen bzw. Fehlmessungen führen kann. Abgesehen davon kann die von ERWEKA angebotene Lösung im Bereich der Zuführung der Tabletten zu dem Rechen störungsanfällig sein bzw. kann sie nicht immer gewährleisten, dass die Tabletten auch in der erforderlichen Position und vereinzelt in den Rechen gelangen. Für einige der einzelnen Messstationen ist es nämlich wesentlich, ob die Tabletten stehend oder liegend bzw. der Länge nach oder quer angeliefert werden. Befinden sich in einer Gabel des Rechens mehr als eine Tablette, kann diese nicht gemessen werden oder es kommt zu einer Ausschussmessung.

Andererseits ist beim Bekannten das Ausscheiden von Tablettenbruchstücken vor dem Durchlaufen der Messstationen nicht sichergestellt, so dass es auch hier zu Fehlmessungen kommen kann.

Insbesondere für unterschiedliche Tablettenformen gibt es diesbezüglich somit noch keine befriedigende, universell anwendbare Technik. Dies führt zur Notwendigkeit von Umbauten für unterschiedliche Tablettenformen bzw. zu einem Zeitverlust im Messbetrieb.

Norbert Krämer aus D-6100 Darmstadt hat bei einem Versuch zur Verbesserung der Situation eine relativ aufwendige Vereinzelungsvorrichtung mit Schikanen für Tabletten geschaffen, die in der DE-C2-3711827 beschrieben ist. Seine Vorrichtung soll auf besonders kurzen Strecken zu einer Vereinzelung der Tabletten führen. Für das Weiterbefördern zu den einzelnen Messstationen bietet diese Beschreibung jedoch keine Lösung. Auch ist das Problem des Lagepositionierens sowie des Ausscheidens von Tablettenbruchstücken durch Krämer nicht gelöst.

Eine andere Herstellerin, Elisabeth Hata, verwendet die Vereinzelungsvorrichtung gemäss Krämer, jedoch ohne dessen Schikanen, was zwar zu einer ausreichenden Vereinzelung bei grösseren Tabletten, jedoch zu einer störanfälligen Vereinzelung bei kleineren Tabletten führt.

Der Erfindung liegt daher die Aufgabe zugrunde, ein Tablettenprüfgerät zu schaffen, das fehlerfrei vereinzelt, die Tabletten lagerichtig positioniert und möglichst universell für die verschiedensten Tablettenformen einsetzbar ist. Beim Betrieb sollen Fehlmessungen vermieden werden und derart insgesamt ein schnelleres Messen als bisher möglich sein.

Gelöst wird diese Aufgabe durch die Kombination an sich bekannter Einzelvorrichtungen in einer erfinderischen Art und Weise. Einzelne dieser Vorrichtungen sind gegenüber den bekannten darüber hinaus noch in erfinderischer Weise verbessert.

Der Grobaufbau besteht dabei erfindungsgemäss aus einer Vereinzelungseinheit, die vorzugsweise als an sich bekannter Vibrationsschneckenförderer "EMSE" mit einer Rutsche aufgebaut ist, einer Orientierungseinheit, die aus einem erfindungsgemäss umgebauten Vibrationslinearförderer aufgebaut ist. Eine besondere Weiterbildung sieht eine Tablettensensor mit erfinderischer Bruchteilfalle und einem speziellen Positionierrechen über einer Transportfläche vor, die den Transport der vorgängig lagerichtig positionierten und auf Ausschuss überprüften Tabletten zu Messstationen bis hin zu einer Restefalle übernimmt.

Anspruch 1 beschreibt den Hauptlösungsgedanken.

Weitere Verbesserungen bzw. Details zu diesem Hauptlösungsgedanken sind in den abhängigen Ansprüchen beschrieben, wobei dort zum Teil auch selbständig und unabhängig vom Hauptlösungsgedanken anwendbare erfinderische Vorrichtungen angegeben sind.

Weitere Details, Vorteile und Anwendungsmöglichkeiten dieser Lösungen sind in der Figurenbeschreibung dargelegt.

Die diesbezüglichen Angaben in dieser Patentanmeldung sind insofern nur beispielhaft zu verstehen, als im Rahmen der Patentansprüche auch andere Detaillösungen als die näher beschriebenen möglich sind.

Es zeigen dabei
- Fig.1: einen Symbolaufbau in der Draufsicht;
- Fig.2: einen Prototypenaufbau in Schrägansicht;

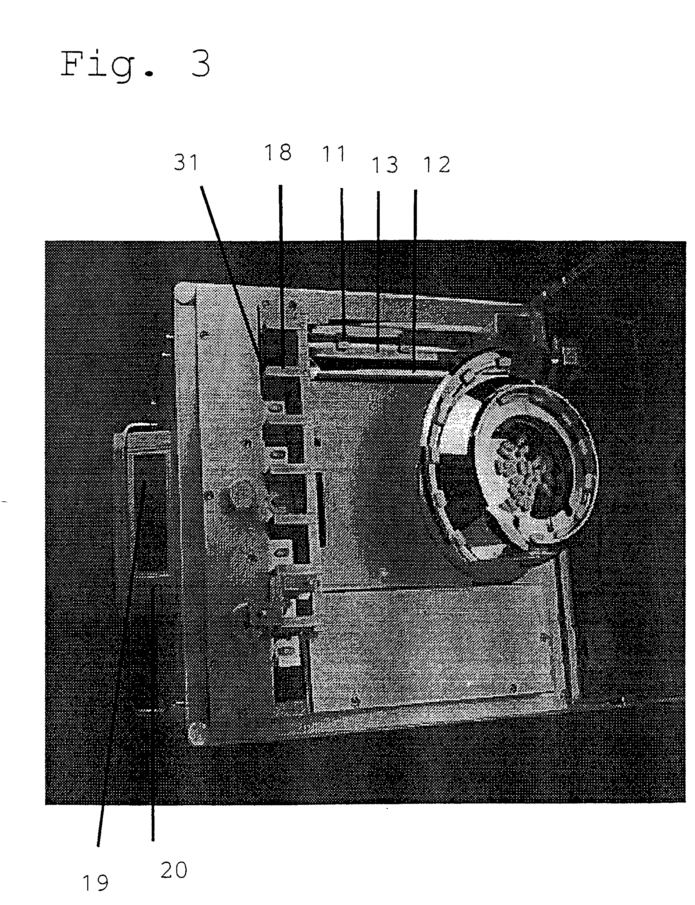

- Fig.3: den Prototypenaufbau in Draufsicht ohne Abdeckung;

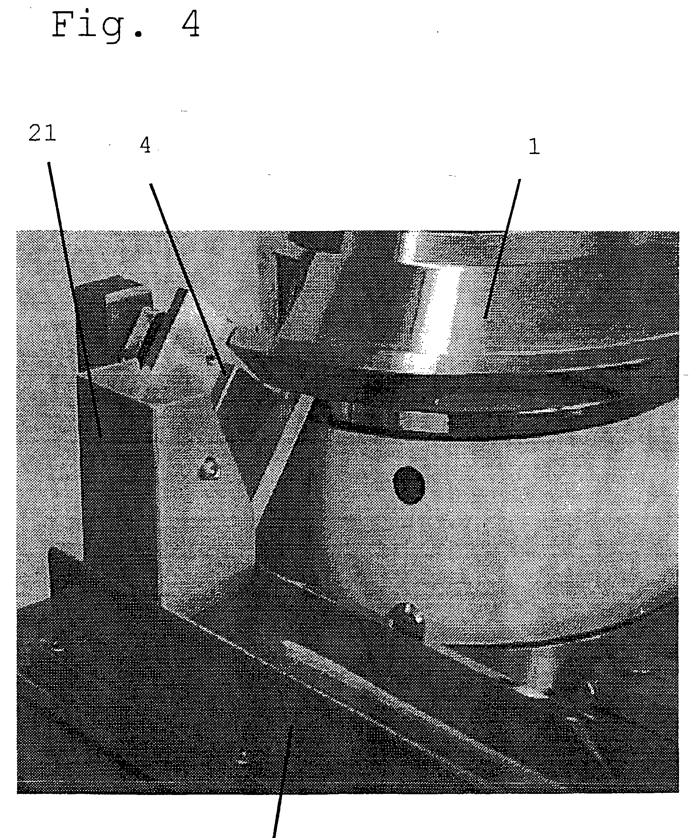

- Fig.4: den Übergang von einem Schneckenförderer zu einem Linearförderer beim Prototypenaufbau;
- Fig.5: einen Rechen am Prototyp;

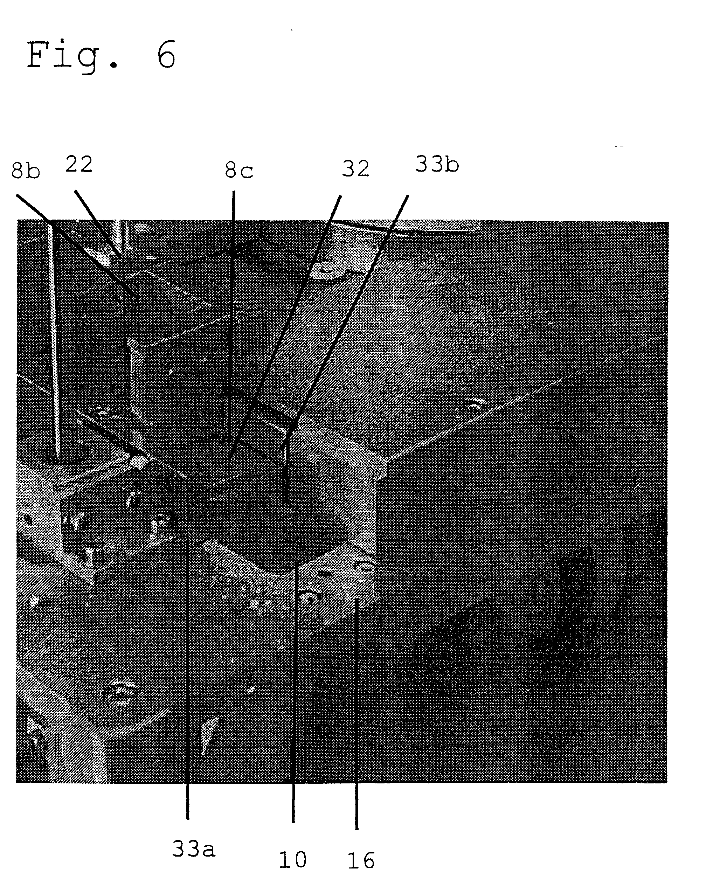

- Fig.6: die Restefalle am Ende des Rechens und
- Fig.7: eine beispielhafte Detailzeichnung eines Tablettensensors in Draufsicht, wobei Fig.7a den geschlossenen Sensor und Fig.7b den geöffneten Sensor mit freiliegender Bruchstückfalle zeigt.

Die Figuren werden zusammenhängend beschrieben, gleiche Teile bedeuten gleiche Bezugszeichen.

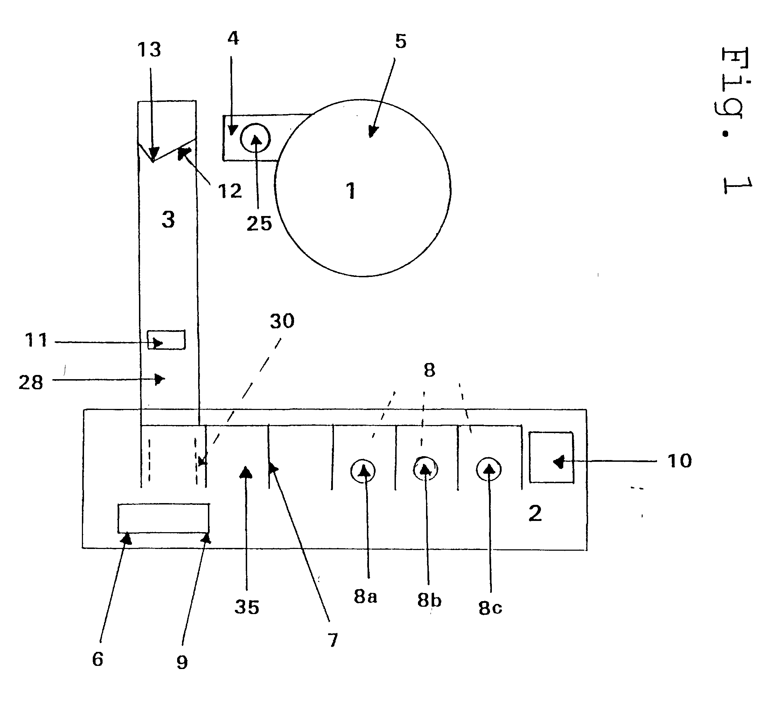

Fig.1 zeigt einen an sich herkömmlichen Schneckenvibrationsförderer 1 mit einem Einfülltrichter 5, dessen Ausgang in eine Rutsche 4 mündet. Der Vibrationsförderer vereinzelt Tabletten und fördert sie entgegen der Schwerkraft in die Rutsche 4. Daran angeschlossen ist eine speziell ausgebildete Orientierungseinheit 3, die auch bei anderen vergleichbaren Vorrichtungen unabhängig eingesetzt werden könnte.

Das Besondere an dieser, auch unabhängig einsetzbaren Einheit, die im wesentlichen aus einem Linearvibrationsförderer besteht, ist eine im Schnitt dargestellte Neigung 12, die zur Folge hat, dass sich die Tabletten - sofern sie eine längliche Struktur aufweisen - in Längsrichtung parallel zur Orientierungseinheit 3 orientieren. Gegen Ende der Orientierungseinheit 3 geht die Neigung 12 erfindungsgemäss in eine annähernd waagerechte Förderfläche 28 über. An der tiefsten Stelle der Orientierungseinheit 3 ist diese mit einem Radius 13 versehen, der stehende Tabletten zum Umfallen bringt, so dass diese mit ihrer Breitseite auf der Neigung 12 bzw. anschliessend auf der Förderfläche 28 liegen.

Für besonders schwierige Tablettenformen können weiters Schikanen 11 angeordnet sein, die einerseits eine Tablette 25 zum Umfallen, andererseits aber auch zu einer bestimmten Längsbzw. Querorientierung zwingen können. Erfindungsgemäss sind diese Schikanen 11 leicht entfernbar bzw. auswechselbar, so dass sie, ohne den Betrieb länger unterbrechen zu müssen,- bei Bedarf gewechselt werden können. Infolge der ausgeklügelten Form der Orientierungseinheit 3 mit Neigung 12 und Radius 13 ist jedoch für die meisten Tablettenformen die Anwendung von Schikanen 11 erlässlich.

Die Orientierungseinheit 3 mündet in einer Positioniereinheit 2, an deren Anfang eine Bruchteilfalle 9 und an deren Ende eine Restefalle 10 angebracht ist. Die Positioniereinheit 2 nimmt Messstationen 8 auf und verfügt über einen seitlich verschiebbaren Positionierrechen 7 mit Zinken 18 für das seitliche Fördern von Tabletten 25 zu den einzelnen Messstationen 8, z.B. einer Wägezelle 8a, einer Dickenmessstelle 8b und einer Bruchfestigkeitsmessstelle 8c.

Erfindungsgemäss sind manche der Zinken 18 aus der Vorschubrichtung etwas mehr als 90° geneigt (vgl. Winkel 30). Dies führt zu einem automatischen Verschiebevektor an den geförderten Tabletten 25 in Richtung der vorderen Stützwand 31, was insofern von Vorteil ist, als sich einzelne der Messstationen 8 mit ihren Aufbauten auch an der Stützwand 31 orientieren und es wichtig ist, dass die Tabletten 25 positionsrichtig bei diesen Aufbauten ankommen. Selbstverständlich liegen im Rahmen der Erfindung auch Möglichkeiten, eine andere Neigung der Zinken 18 z.B. in die andere Richtung vorzusehen, sollten die Tabletten 25 bei ihrem Weitertransport auch noch von der Stützwand 31 wegbewegt werden. Diese Möglichkeit umfasst auch einstellbare Neigungen an den Zinken 18, um diese sich ändernden Gegebenheiten anpassen zu können.

Gemäss einer besonderen Ausgestaltung verfügen die Zinken 18 an ihrer Unterseite über Bürsten 34, einen Filzstreifen o.dgl., die der Staubfreimachung (Tablettenstaub) der Führungsbahn dienen. Durch die Bewegung des Rechens 7 wird dies optimal gleichzeitig mit der Tablettenförderung bewerkstelligt. Der Rechen 7 vollführt eine Seitverschiebung, anschliessend wird er angehoben und denselben Seitverschiebeweg zurückgesetzt, um ihn dann wieder abzusinken und die nächste Seitverschiebung durchführen zu können.

Die letzte Messstation 8c verfügt als erfindungsgemässe Besonderheit über eine längliche Vertiefung 32, die eine letzte Längspositionierung von länglichen Tabletten erlaubt, bevor diese durch die Spannbacken 33a,b erfasst werden.

Gemäss einer besonderen Ausgestaltung der Erfindung sind Spannbacken 33 und die letzte Zinke 18 so ausgebildet, dass angebackene Tablettenreste an den Backen 33 und/oder an der Führungsebene beseitigt werden. Dadurch werden die Messergebnisse optimiert.

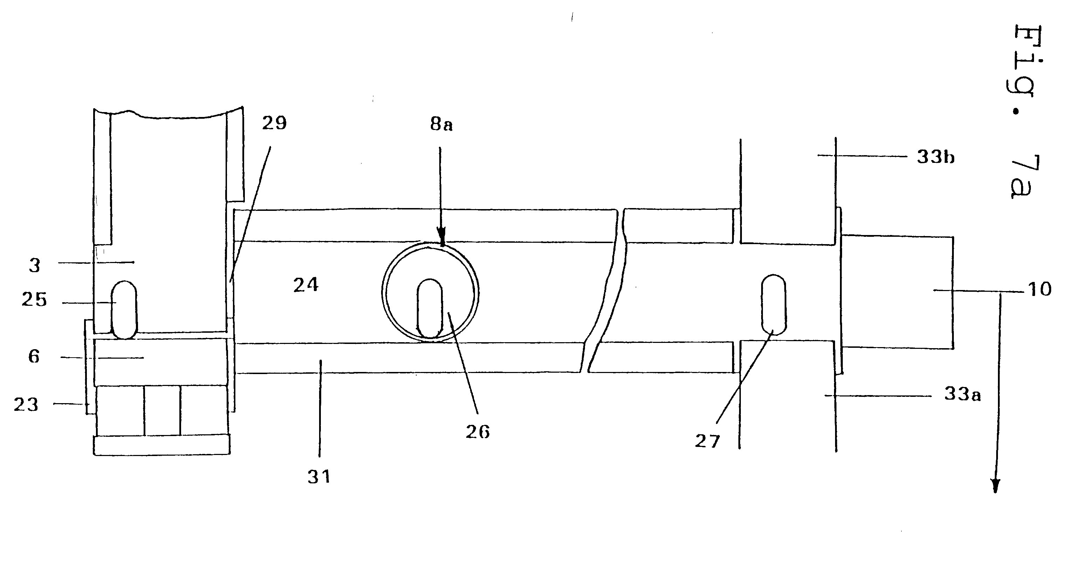

Die erste Bruchteilfalle 9 ist erfindungsgemäss mit einem Tablettensensor 6 abgedeckt, der beim Auftreffen einer Tablette 25 den Transportrechen 7 triggert und gleichzeitig einen Spalt von z.B. 3mm freigibt (vgl. Fig.7a), um gleichzeitig mitgelieferte Tablettenbruchstücke nach unten wegfallen zu lassen.

Im Falle einer unzulässigen Doppellieferung kann der erfindungsgemässe Sensor 6 dieses ebenso detektieren und die Bruchstückfalle 9 öffnen, um weitere Fehler zu verhindern.

"Sensor" im Sinne der Erfindung umfasst auch einen reinen, freigebbaren "Anschlag" für Bruchstückreste o.dgl., sofern dieser in Abhängigkeit von den angeförderten Tabletten oder - resten gesteuert ist. Insbesondere kann der Sensor auch eine Lichtschranke, gegebenenfalls mit abbildender Optik umfassen. Bei einem Ausführungsbeispiel ist der Sensor jedoch als fahrbarer, mit einer Piezoschaltfläche versehener Schieber ausgebildet.

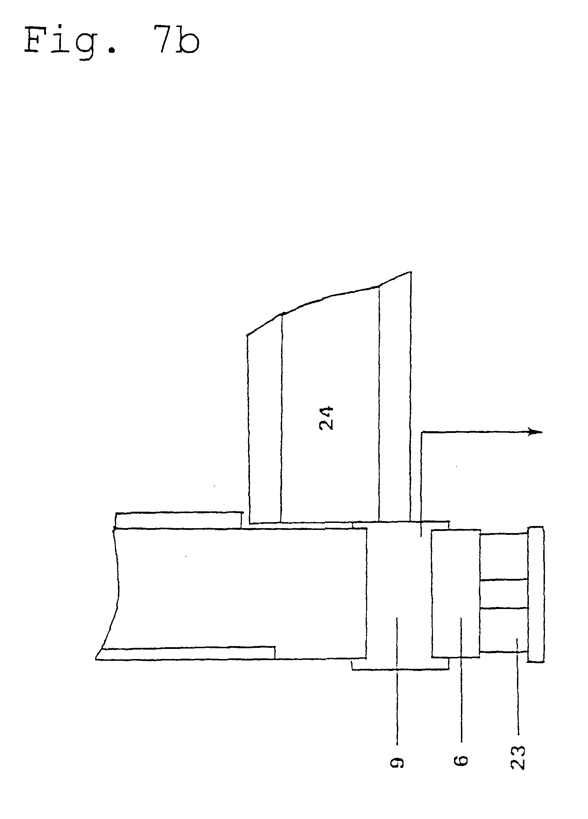

Gemäss einer besonderen Variante des Sensors 6 verfügt dieser über-an sich bekannte, nicht näher dargestellte z.B. lichtoptische Messsensoren, die die Vollständigkeit einer Tablette 25 erkennen und im Negativfall den Spalt so weit öffnen, dass auch grössere Bruchstücke durch die Bruchstückfalle 9 nach unten wegfallen können (vgl. Fig.7b).

Eine mögliche Sensoreinstellung wäre z.B.: Wenn eine Tablette mit 6mm Durchmesser ankommt, fährt er kurz auf die Spaltbreite von 2-3mm zurück und dann wieder so weit vor, dass die Tablette 1-2mm vor der Stützfläche der Stützwand 31 zu liegen kommt. Durch das Verbreitern des Spaltes fallen allfällige Bruchstückreste weg, durch das Zurückschieben der Tablette 25 wird diese für bestimmte Messstellen positioniert.

Selbstverständlich liegen im Rahmen der Erfindung auch Varianten mit einer besonderen Softwaresteuerung, die es beispielsweise ermöglicht, den Schneckenvibrationsförderer 1 und die Orientierungseinheit 3 von überzähligen Tabletten aus einer Messserie freizufördern, indem nach einer bestimmten, durch den Sensor erkannten und mittels Rechen 7 weitergeförderten Tablettenmenge die Bruchstückfalle 9 durch den Sensor freigegeben wird.

Die Fallen 9 und 10 münden in je eine Box 15 an der Vorderseite des Gerätes, so dass diese bedienerfreundlich entsorgt werden können, was auch ein besonderer Vorteil dieser Variante der Erfindung ist.

Eine durchsichtige Abdeckung 14 schützt das Gerät vor Staub und unerwünschtem Zugriff, ohne die Funktionen bzw. den funktionsrichtigen Ablauf unsichtbar zu machen.

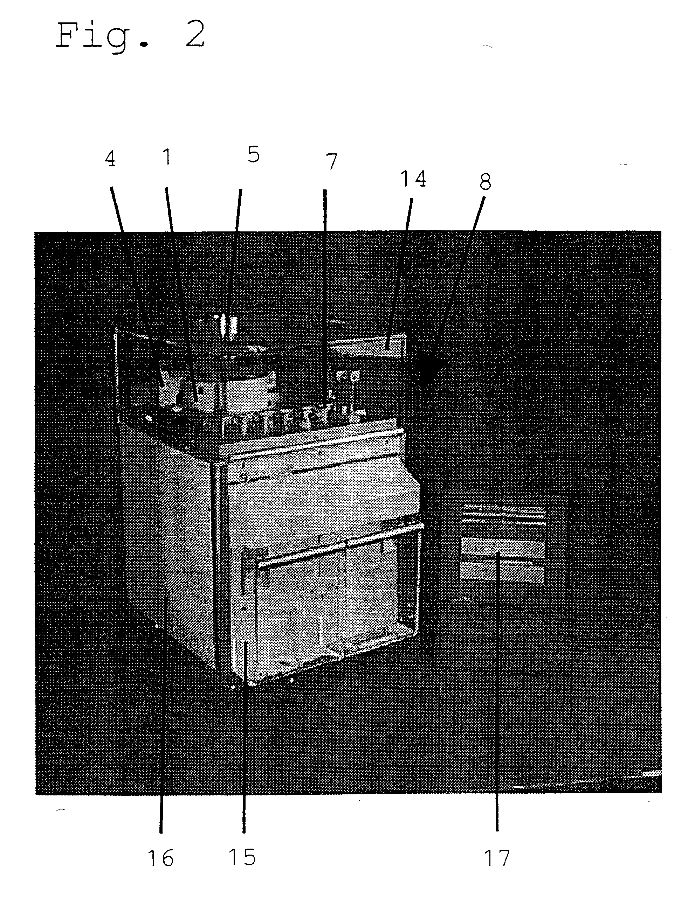

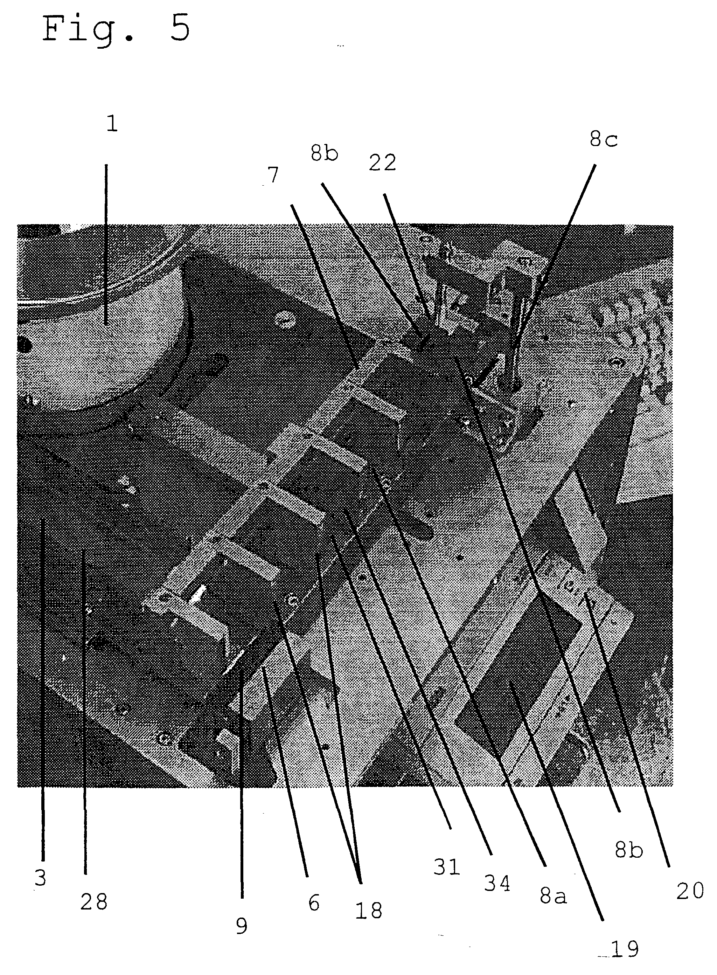

Ein gegebenenfalls abdeckbares (Fig.2) Display 19 und eine Eingabeeinheit 20 (Fig.5) ermöglichen das Steuern des Gerätes, sofern es nicht ohnedies mittels Computer 17 (Fig.2) angesteuert ist.

Die Antriebe und elektrischen Steuerungen des Gerätes sind im Inneren eines Gestells 16 untergebracht, das die beschriebenen Teile trägt.

Gemäss einer besonderen Ausgestaltung der Erfindung sind alle Laufflächen bzw. Gleitflächen, die durch die Tabletten 25 berührt werden können reibungsarm veredelt, z.B. elektrolytisch poliert, hochglanzverchromt oder poliert.

Gemäss einer weiteren Ausgestaltung der Erfindung ist der Wiegeteller 26 der Wägezelle 8a so angeordnet, dass seine Achse quer zur Transportrichtung identisch ist mit der Querachse einer angeförderten Tablette, während die Mittelachse einer Tablette parallel zur Stützwand 31 mit der Mittelachse des Wiegetellers 26 nicht zwingend übereinstimmt. Bevorzugt liegt die Tablette so nahe wie möglich an der Stützwand 31. Gerade so, dass sie noch mit vollem Umfang auf dem Wiegeteller 26 liegt. Dies kann u.a. auch dadurch erreicht werden, dass der Sensor 6 mit seiner Anschlagkante etwa 1,5mm vor der Stützfläche der Stützwand 31 zu liegen kommt - gegebenenfalls nach vorn geschoben wird.

Weitere Angaben zu den Figuren können der Bezugszeichenliste entnommen werden.

Durch die bisher angegebenen Ausbildungen ist die Erfindung nicht eingeschränkt. Sie umfasst einen breitern Rahmen:

Im Sinne der Erfindung bedeuten "Tabletten" auch "Kapseln" o.dgl.

"Schikanen" umfassen auch "Abweiser" und können gegebenenfalls auch durch Luftdüsen o.dgl. gebildet sein.

Luft- oder Vakuumdüsen können im übrigen, wie nicht näher dargestellt, an mehreren Orten, z.B. zum Zwecke der automatischen Reinigung, angebracht sein, so wie Pressluft auch zum Steuern der mechanischen Bauteile vorgesehen sein kann.

Die Orientierungseinheit 3 kann gemäss einer weiteren Variante auch mit einer Weiche oder einem Sensorausstosser versehen sein, die bzw. der unabhängig vom Sensor 6 eine Steuerung von Tabletten beeinflussen kann. Z.B. kann dadurch eine noch nicht befriedigende Vereinzelung überwacht und korrigiert werden. Andererseits könnten auch mehrere Tabletten unmittelbar hintereinander in der Orientierungseinheit anstehen und vereinzelt durch Öffnen einer solchen Weiche weitergefördert werden. In der Regel sollen jedoch die Tabletten vereinzelt angeliefert werden.

Als weitere Variante ist es denkbar, die Rutsche 4 drehbar zu gestalten, so dass eine weitere Leerfördermöglichkeit in einen weiteren, nicht dargestellten Behälter möglich ist. Durch die erfindungsgemässen Eigenschaften des Sensors wird dies jedoch in der Regel überflüssig sein.

Gemäss einer weiteren besonderen Ausgestaltung der Erfindung ist der Positionier- bzw. Transportrechen mit seitlich verstell- bzw. verschwenkbaren Zinken 18 versehen, die in Abhängigkeit von der Breite von Tabletten so eingestellt werden, dass Tabletten, deren Längserstreckung senkrecht zur Transportrichtung liegt in exakt dieser Position verschoben werden. Die Zinken 18 des Transportrechens liegen somit seitlich an den Tabletten an, sobald diese in Transportrichtung verschoben werden. Nach dem Verschub der Tabletten öffnen die Zinken geringfügig, werden sodann wie bei der oben beschriebenen Variante abgehoben und zurückversetzt, um die nächsten Tabletten zu erfassen und weiter zu schieben. In Abhängigkeit vom Tablettenaufbau wird diese Zinkenbewegung automatisch gesteuert. Diese besondere Ausgestaltung hat den Vorteil, dass selbst bei etwas schief ankommenden Tabletten, diese richtig positioniert werden können, und dass darüber hinaus beim Transport mittels Transportrechen die Tabletten nicht unabsichtlich aus ihrer Lage geschwenkt werden können. Das Öffnen der Zinken kurz vor dem Abheben des Transportrechens ist erfindungsgemäss deshalb von Vorteil, weil dadurch vermieden wird, dass Tabletten unabsichtlich in Folge Reibung an den Zinkenwänden aufgekantet werden.

Eine weitere besondere Ausgestaltung ist im Bereich der Bruchfestigkeitsmessstelle 8c vorgesehen. Anstelle der Vertiefung 32 kann dort auch ein auswechselbarer Auflageteller vorgesehen sein, der eine vergleichbare Vertiefung ausgebildet hat, wobei die unterschiedlichen Auflageteller unterschiedliche Vertiefungen aufweisen. Tabletten werden nämlich in unterschiedlichen Grössen erzeugt, so dass es für die Funktion der Vorrichtung verbessert ist, wenn die Vertiefung 32 an die Tablettengrösse angepasst ist. Ein zu schmale Vertiefung 32 würde keinen Positioniereffekt zeigen, da die zu breite Tablette gar nicht einsinken würde. Eine kleinere Tablette wiederum, könnte in der Vertiefung 32 zu tief einsinken, so dass die Pressbacken keinen optimalen Angriff an die Tablette finden.

### Bezugszeichenliste

- 1.: Vereinzelungseinheit bzw. Schneckenvibrationsförderer
- 2.: Positioniereinheit
- 3.: Orientierungseinheit mit Vibrationslinearförderer
- 4.: Rutsche
- 5.: Einfülltrichter
- 6.: Tablettensensor
- 7.: Positionierrechen bzw. Transportrechen
- 8.: Messstationen
- 8a.: Wägezelle
- 8b.: Dickenmessstelle
- 8c.: Bruchfestigkeitsmessstelle
- 9.: Bruchteilfalle
- 10.: Restefalle
- 11.: Schikanen
- 12.: Neigung; kann gegebenenfalls auch einstellbar sein.
- 13: Radius; kann gegebenenfalls einstell- oder austauschbar ausgebildet sein.
- 14.: Haube
- 15.: Box
- 16.: Gestell
- 17.: Computer
- 18.: Zinken
- 19.: abdeckbares Display
- 20.: Eingabefeld/Eingabeeinheit
- 21.: Rutschenabdeckung
- 22.: Messstössel
- 23.: Antrieb/Bürste
- 24.: Förderbahn
- 25.: Tablette
- 26.: Wiegeteller
- 27.: Positionierausnehmung
- 28.: annähernd waagrechte Förderfläche
- 29.: Spalt
- 30.: Winkel
- 31.: Stützwand
- 32.: Vertiefung, Positionierausnehmung
- 33.: Spannbacken
- 34.: Bürsten
- 35.: Förderfläche

## Patentansprüche

1. Tablettenprüfgerät mit wenigstens einer Messstation (8), mit einer Vorrichtung zum Anfördern und Vereinzeln von Tabletten (25), mit einer anschliessenden Orientierungseinheit (2) und mit einem daran anschliessenden Positionierrechen (7) über einer Transportfläche (35) für den Transport der Tabletten (25) zu der Messstation (8), dadurch gekennzeichnet, dass die Orientierungseinheit (2) aus einem Vibrationslinearförderer (3) aufgebaut ist, der über einen im Querschnitt L-förmigen Aufbau mit einer Neigung (12) und einem Radius (13) zwischen den beiden Schenkeln des Ls Verfügt, wobei er vorzugsweise im Bereich seiner Mündung beim Positionierrechen (7) in eine horizontale Förderfläche (28) übergeht.

2. Tablettenprüfgerät nach Anspruch 1, dadurch gekennzeichnet, dass am Eingang des Positionierrechens (7) ein Tablettensensor (6) angeordnet ist, um ankommende Tabletten (25) zu detektieren und den Positionierrechen (7) zu triggern, und dass die Vorrichtung zum Anfördern insbesondere als Schneckenvibrationsförderer (1) ausgebildet ist.

3. Tablettenprüfgerät nach Anspruch 1 oder 2, dadurch gekennzeichnet, dass der Tablettensensor (6) einen motorisch antreibbaren Schieber umfasst, der eine Bruchstückfalle 9 grogramm- und/oder sensorgesteuert wahlweise vollständig oder teilweise freigeb- oder schliessbar macht, wobei der Sensor (6) vorzugsweise über eine Lichtschranke und/oder über einen Anlaufpiezotaster aufweist, welch' letztere ankommende Tabletten detektierbar machen.

4. Tablettenprüfgerät nach einem der vorhergehenden Ansprüche, dadurch gekennzeichnet, dass der Vibrationslinearförderer (2) an seiner Neigung (12) und/oder an seiner Förderfläche (28) über vorzugsweise austauschbare Schikanen (11) verfügt, und/oder dass seine Neigung (12) und/oder sein Radius (13) einstellbar sind.

5. Tablettenprüfgerät nach einem der vorhergehenden Ansprüche, dadurch gekennzeichnet, dass zwischen dem Vibrationsschneckenförderer (1) und der Orientierungseinheit (3) eine Rutsche (4) angeordnet ist, wobei diese einen Staubabscheidungsspalt bietet oder aufweist, und dass die Rutsche gegebenenfalls einstell- bzw. motorisch schwenkbar ist, um eine Leerförderung des Vibrationsschneckenförderers (1) zu ermöglichen.

6. Tablettenprüfgerät nach einem der vorhergehenden Ansprüche, dadurch gekennzeichnet, dass die Transportfläche (35) im Schnitt L- oder U-förmig aufgebaut ist, wobei ein senkrechter Schenkel eine Stützwand (31) bildet, entlang der die Messstationen (8) - in die Förderfläche (35) integriert - angeordnet sind, und/oder dass am Ende der Förderfläche (35) eine Restefalle (10) vorgesehen ist.

7. Tablettenprüfgerät nach einem der vorhergehenden Ansprüche, dadurch gekennzeichnet, dass eine Messstation (8c) als Bruchfestigkeitsmessstation ausgebildet ist und über Spannbacken (33) verfügt, die quer zur Transportrichtung der Tabletten (25) etwa parallel zur Förderfläche (35) verschiebbar sind wobei die Förderfläche (35) im Bereich zwischen den Spannbacken (33) vorzugsweise über eine Positionierausnehmung (32) verfügt.

8. Tablettenprüfgerät nach einem der vorhergehenden Ansprüche, dadurch gekennzeichnet, dass der Transportrechen (7) über Zinken (18) verfügt, die in Draufsicht in einem Winkel (30) von ungleich 90° aus der Transportrichtung geneigt sind, und/oder dass die Zinken (18) an ihrer der Förderfläche (35) zugewandten Seite Bürsten, Filzstreifen o.dgl. aufweisen, und/oder dass die Zinken (18) so ausgebildet sind, dass sie bei einer Förderbewegung oder bei einer Rückstellbewegung Teile der Messstationen (z.B. Spannbacken (33)) reinigen.

9. Tablettenprüfgerät nach einem der vorhergehenden Ansprüche, dadurch gekennzeichnet, dass die Zinken (18) des Transportrechens (7). - vorzugsweise in Abhängigkeit von Tablettendimensionen automatisch - einstell- bzw. verschwenkbar sind.

10. Tablettenprüfgerät nach einem der vorhergehenden Ansprüche, dadurch gekennzeichnet, dass im Bereich der Bruchfestigkeitsmessstation (8c) auswechselbare - vorzugsweise in Abhängigkeit von der Tablettendimension automatisch auswechselbare - Auflageteller vorgesehen sind.

11. Tablettenprüfgerät nach einem der vorhergehenden Ansprüche, dadurch gekennzeichnet, dass den Fallen (6,9,10) Boxen (15) zugeordnet sind, die vorzugsweise an der Vorderseite des das Gerät tragenden Gestells (16) angeordnet sind.

12. Tablettenprüfgerät nach einem der vorhergehenden Ansprüche, dadurch gekennzeichnet, dass alle tablettenberührenden Flächen oberflächenveredelt, poliert o.dgl. sind.

13. Tablettenprüfgerät mit wenigstens einer Messstation (8), mit einer Vorrichtung zum Anfördern und Vereinzeln von Tabletten (25), mit einer anschliessenden Orientierungseinheit (2) und mit einem daran anschliessenden Positionierrechen (7) über einer Transportfläche (35) für den Transport der Tabletten (25) zu der Messstation (8) und mit einem Tablettensensor, der die ankommenden Tabletten detektiert und den Positionierrechen triggert, **dadurch gekennzeichnet**, dass die Orientierungseinheit (2) aus einem Vibrationslinearförderer (3) aufgebaut ist, der über einen im Querschnitt L-förmigen Aufbau mit einer Neigung (12) und einem Radius (13) zwischen den beiden Schenkeln des Ls verfügt, wobei er vorzugsweise im Bereich seiner Mündung beim Positionierrechen (7) in eine horizontale Förderfläche (28) übergeht.

14. Tablettenprüfgerät nach Anspruch 13, **dadurch gekennzeichnet, dass der** Tablettensensor (6) am Eingang des Positionierrechens angeordnet ist, und dass die Vorrichtung zum Anfordern insbesondere als Schneckenvibrationsförderer (1) ausgebildet ist.

## Claims

1. A tablet tester with at least one measuring station (8), having an apparatus for conveying and isolating tablets (25), having a downstream orientation unit (2) and having a positioning rake (7) connected thereto above a conveying surface (35) for transporting the tablets (25) to the measuring station (8), wherein the orientation unit (2) comprises a linear vibratory conveyor (3) which has a structure L-shaped in cross-section and having an inclination (12) and a radius (13) between the two limbs of the L, which conveyor preferably becomes a horizontal conveying surface (28) in the region where it enters the positioning rake (7).

2. A tablet tester as claimed in claim 1, wherein a tablet sensor (6) is arranged at the entrance of the positioning rake (7) in order to detect incoming tablets (25) and to trigger the positioning rake (7), and wherein the apparatus for transporting is in particular in the form of a vibratory screw conveyor (1).

3. A tablet tester as claimed in claim 1 or 2, wherein the tablet sensor (6) comprises a slide which can be driven by a motor and which makes a fragment trap 9 either completely or partially openable or closable by program control and/or sensor control, the sensor (6) preferably having a light barrier and/or a piezoelectric surface switch, which latter makes incoming tablets detectable.

4. A tablet tester as claimed in any of the preceding claims, wherein the linear vibratory conveyor (2) has preferably interchangeable baffles (11) on its inclination (12) and/or on its conveying surface (28), and/or wherein its inclination (12) and/or its radius (13) are adjustable.

5. A tablet tester as claimed in any of the preceding claims, wherein a chute (4) is arranged between the vibratory screw conveyor (1) and the orientation unit (3), said chute offering or having a dust separation gap, and wherein the chute optionally is adjustable or is pivotable by means of a motor, in order to permit emptying of the vibratory screw conveyor (1).

6. A tablet tester as claimed in any of the preceding claims, wherein the transport surface (35) is L- or U-shaped in section, a perpendicular limb forming a support wall (31) along which the measuring stations (8) - integrated in the conveying surface (35) - are arranged, and/or wherein a residue trap (10) is provided at the end of the conveying surface (35).

7. A tablet tester as claimed in any of the preceding claims, wherein a measuring station (8c) is in the form of a hardness measuring station and has clamping jaws (33) which are displaceable crosswise to the transport direction of the tablets (25), approximately parallel to the conveying surface (35), the conveying surface (35) preferably having a positioning recess (32) in the region between the clamping jaws (33).

8. A tablet tester as claimed in any of the preceding claims, wherein the transport rake (7) has prongs (18) which, in plan view, are inclined relative to the transport direction by an angle (30) which is not equal to 90°, and/or wherein the prongs (18) have brushes, felt strips or the like on their side facing the conveying surface (35), and/or wherein the prongs (18) are formed in such a way that they clean parts of the measuring stations (for example, clamping jaws (33)) during a conveying movement or during a resetting movement.

9. A tablet tester as claimed in any of the preceding claims, wherein the prongs (18) of the transport rake (7) are adjustable or pivotable - preferably automatically as a function of tablet dimensions

10. A tablet tester as claimed in any of the preceding claims, wherein interchangeable support disks - preferably automatically interchangeable as a function of the tablet dimension - are provided in the region of the hardness measuring station (8c).

11. A tablet tester as claimed in any of the preceding claims, wherein boxes (15), which are preferably arranged on the front of the rack (16) carrying the apparatus, are assigned to the traps (6, 9, 10).

12. A tablet tester as claimed in any of the preceding claims, wherein all surfaces which come into contact with tablets are surface-treated, polished or the like.

13. A tablet tester with at least one measuring station (8), having an apparatus for conveying and isolating tablets (25), having a downstream orientation unit (2) and having a positioning rake (7) connected thereto above a conveying surface (35) for transporting the tablets (25) to the measuring station (8) and having a tablet sensor which detects the arriving tablets and triggers the positioning rake, wherein the orientation unit (2) comprises a linear vibratory conveyor (3) which has a structure L-shaped in cross-section and having an inclination (12) and a radius (13) between the two limbs of the L, which conveyor preferably becomes a horizontal conveying surface (28) in the region where it enters the positioning rake (7).

14. A tablet tester as claimed in claim 13, wherein the tablet sensor (6) is arranged at the entrance of the positioning rake and wherein the apparatus for transporting is in particular in the form of a vibratory screw conveyor (1).

## Revendications

1. Vérificateur de produits pastillés, avec au moins un poste de mesure (8), avec un dispositif pour amener et individualiser des produits pastillés (25), avec une unité d'orientation (2) y rattaché, et un râteau positionneur (7) s'y rattachant via une surface de transport (35) pour le transport des produits pastillés (25) vers le poste de mesure (8), caractérisé en ce que l'unité d'orientation (2) est constituée d'un convoyeur linéaire vibrant (3) possédant, en coupe transversale, une structure de L avec une pente (12) et un rayon (13) entre les deux côtés du L, en rejoignant de préférence une face de transport horizontale (28) dans la zone de son embouchure au niveau du râteau positionneur (7).

2. Vérificateur de produits pastillés selon la revendication 1, caractérisé en ce que l'entrée du râteau positionneur (7) se voit associer un détecteur de produits pastillés (6), pour détecter des produits pastillés (25) arrivants et activer le râteau positionneur (7), et en ce que le dispositif d'amenée est conçu en particulier sous la forme d'un convoyeur vibrant à vis sans fin (1).

3. Vérificateur de produits pastillés selon la revendication 1 ou 2, caractérisé en ce que le détecteur de produits pastillés (6) comprend un tiroir pouvant être entraîné par moteur et qui peut rendre une trappe à fragments (9 optionnellement libérable ou refermable en totalité ou en partie, sous la commande d'un programme et/ou d'un capteur, le capteur (6) présentant de préférence un barrage photoélectrique et/ou une sonde piézoélectrique de démarrage qui rendent détectables les derniers produits pastillés arrivants.

4. Vérificateur de produits pastillés selon l'une des revendications précédentes, caractérisé en ce que le convoyeur linéaire vibrant (2) présente, au niveau de sa pente (12) et/ou de sa face de transport (28), des chicanes (11) de préférence interchangeables, et/ou en ce que sa pente (12) et/ou son rayon (13) sont ajustables.

5. Vérificateur de produits pastillés selon l'une des revendications précédentes, caractérisé en ce qu'on a disposé entre le convoyeur vibrant à vis sans fin (1) et l'unité d'orientation (3) une glissière (4), cette dernière offrant ou présentant une fente de séparation de poussières, et en ce que la glissière est le cas échéant ajustable ou pivotante par moteur, pour permettre un vidage du convoyeur vibrant à vis sans fin (1).

6. Vérificateur de produits pastillés selon l'une des revendications précédentes, caractérisé en ce que la face de transport (35) présente, en coupe, une forme de L ou de U, un côté vertical formant une paroi de soutien (31) le long de laquelle les postes de mesure (8) sont intégrés dans la surface de transport (35), et/ou en ce qu'on a prévu une trappe à résidus (10) à la fin de la surface de transport (35).

7. Vérificateur de produits pastillés selon l'une des revendications qui précèdent, caractérisé en ce qu'un poste de mesure (8c) est conçu comme poste de mesure de résistance à la rupture et présente des mordaches de serrage (33) déplaçables transversalement au sens de transport des produits pastillés (25) et à peu près parallèlement à la surface de transport (35), la surface de transport (35) présentant de préférence, dans la zone située entre les mordaches de serrage (33) un creux de positionnement (32).

8. Vérificateur de produits pastillés selon l'une des revendications qui précèdent, caractérisé en ce que le râteau de transport (7) présente des dents (18) qui en vue du dessus sont inclinées d'un angle (30) irrégulier de 90° par rapport au sens du transport, et/ou en ce que les dents (18) présentent sur leur côté orienté vers la surface de transport (35), des brosses, des bandes de feutre, ou similaires, et/ou en ce que les dents (18) sont conçues de façon telle qu'elles nettoient des parties des postes de mesure (p.ex. mordaches de serrage (33)) lors d'un mouvement de convoyage ou de recul.

9. Vérificateur de produits pastillés selon l'une des revendications qui précèdent, caractérisé en ce que les dents (18) du râteau positionneur (7) sont ajustables ou pivotantes automatiquement de préférence en fonction des dimensions des produits pastillés.

10. Vérificateur de produits pastillés selon l'une des revendications précédentes, caractérisé en ce qu'on a prévu dans la zone du poste de mesure de résistance à la rupture (8c) des assiettes d'appui interchangeables, de préférence interchangeables automatiquement en fonction de la dimension des produits pastillés.

11. Vérificateur de produits pastillés selon l'une des revendications précédentes, caractérisé en ce que les trappes (6, 9, 10) se voient associer des boxes (15) qui sont de préférence disposés sur la face antérieure du châssis (16) supportant l'appareil.

12. Vérificateur de produits pastillés selon l'une des revendications précédentes, caractérisé en ce que toutes les surfaces en contact avec les produits pastillés sont anoblies superficiellement, polies ou soumises à un traitement équivalent.

13. Vérificateur de produits pastillés comportant au moins un poste de mesure (8), un dispositif pour amener et individualiser des produits pastillés (25), une unité d'orientation (2) y rattachée et un râteau positionneur (7) s'y rattachant via une surface de transport (35) pour le transport des produits pastillés (25) vers le poste de mesure (8), et un détecteur de produits pastillés, qui détecte les produits pastillés arrivants et active le râteau positionneur, caractérisé en ce que l'unité d'orientation (2) est constituée d'un convoyeur linéaire vibrant (3) présentant une structure en forme de L en coupe transversale avec une pente (12) et un rayon (13) entre les deux côtés du L, en rejoignant une surface de transport horizontale (28) dans la zone de son embouchure au niveau du râteau positionneur (7).

14. Vérificateur de produits pastillés selon la revendication 13, caractérisé en ce que le détecteur de produits pastillés (6) est disposé à l'entrée du râteau positionneur et en ce que le dispositif d'amenée est conçu en particulier comme convoyeur vibrant à vis sans fin (1).
